# EUROPEAN PATENT APPLICATION

(11) **EP 4 052 701 A1**
(43) Date of publication of application: **07.09.2022**
(21) Application number: 20882746.9
(22) Date of filing: 22.10.2020
(51) Int. Cl.: A61K 31/045, A23L 2/52, A23L 33/105, A61K 8/63, A61K 8/9789, A61K 9/08, A61K 9/16, A61K 9/20, A61K 36/48, A61P 1/10, A61P 1/14, A61P 1/16, A61P 3/04, A61P 3/06, A61P 3/10, A61P 21/06, A61P 43/00, C07J 63/00

(54) **COMPOSITION FOR TGR5 ACTIVATION**

(30) Priority: 30.10.2019 JP 2019197377
(71) Applicant: Suntory Holdings Limited, Osaka 530-8203 (JP)
(72) Inventor: KASAJIMA, Naoki, Soraku-gun, Kyoto 619-0284 (JP); FUNAKI, Ayuta, Soraku-gun, Kyoto 619-0284 (JP)
(74) Representative: Vossius & Partner Patentanwälte Rechtsanwälte mbB
(86) International application number: PCT/JP2020/039684
(87) International publication number: WO 2021/085291

(57) **Abstract**

The present invention aims to provide a composition for activating TGR5 containing a substance having a TGR5-activating action and high selectivity to TGR5 as an active ingredient, and a method of activating TGR5 using a substance having a TGR5-activating action and high selectivity to TGR5. The present invention relates to, for example, a composition for activating TGR5, the composition containing at least one soyasapogenol as an active ingredient.

## Description

### TECHNICAL FIELD

The present invention relates to a composition for activating TGR5. The present invention also relates to a method of activating TGR5, for example.

### BACKGROUND ART

Transmembrane G protein-coupled Receptor 5 (TGR5) is a G protein-coupled bile acid receptor and is expressed in many tissues including skeletal muscle and brown adipose tissue. Bile acid is known as an in vivo ligand for TGR5. Bile acid in blood activates intracellular signals by binding to TGR5, resulting in anti-obesity and hypoglycemic actions, for example. According to Patent Literature 1, for example, soybean saponin has a TGR5-activating action.

### CITATION LIST

### - Patent Literature

Patent Literature 1: JP 2016-27012 A

### SUMMARY OF INVENTION

### - Technical Problem

Desirably, agonist compounds for receptors are highly selective to target receptors. An agonist compound with a low selectivity easily binds to non-target receptors, which tends to result in problems such as side effects and difficulty in sufficiently obtaining a desired effect. TGR5 is desirably a substance having a TGR5-activating action and highly selective to TGR5. Patent Literature 1 is silent about selectivity of a TGR5 activator to TGR5.

The present invention aims to provide a composition for activating TGR5 containing a substance having a TGR5-activating action and high selectivity to TGR5 as an active ingredient. The present invention also aims to provide a method of activating TGR5 using a substance having a TGR5-activating action and high selectivity to TGR5.

### - Solution to Problem

As a result of extensive studies in view of the above issues, the present inventors found that soyasapogenols have a TGR5-activating action and are highly selective to TGR5.

Specifically, the present invention relates to, for example, the following composition for activating TGR5, but it is not limited thereto.
(1) A composition for activating TGR5 containing at least one soyasapogenol as an active ingredient.
(2) The composition for activating TGR5 according to (1) above, wherein the at least one soyasapogenol is at least one of soyasapogenol A and soyasapogenol B.
(3) The composition for activating TGR5 according to (1) or (2) above, wherein the composition is a food or beverage, a cosmetic product, or a quasi-pharmaceutical product.
(4) Use of at least one soyasapogenol for activating TGR5.
(5) A method of activating TGR5, including administering at least one soyasapogenol.

### - Advantageous Effects of Invention

The present invention provides, for example, a composition for activating TGR5 containing a substance having a TGR5-activating action and high selectivity to TGR5 as an active ingredient. The composition for activating TGR5 of the present invention, which contains at least one soyasapogenol as an active ingredient, has a TGR5-activating action and is highly selective to TGR5. Soyasapogenols used in the present invention are components that have a long history of consumption as food or beverages ingredients. Advantageously, soyasapogenols can be ingested continuously with high safety. The present invention can also provide a method of activating TGR5 using a substance highly selective to TGR5.

### DESCRIPTION OF EMBODIMENTS

The composition for activating TGR5 of the present invention contains at least one soyasapogenol as an active ingredient.

In the present invention, transmembrane G protein-coupled receptor 5 (TGR5) is preferably a human TGR5 (hTGR5).

Soyasapogenols (which can also be referred to as soyasapogenol compounds) have an action to activate TGR5 (agonist action). Soyasapogenols have an action to activate TGR5 and increase the intracellular cAMP concentration. Soyasapogenols are highly selective to TGR5.

Soyasapogenols are compounds found in Leguminosae plants such as soybean (binomial name: *Glycine max*). Soyasapogenols are one type of triterpenoids. In the present invention, at least one soyasapogenol may include only one compound or two or more compounds. In one embodiment, the composition for activating TGR5 of the present invention may contain only at least one soyasapogenol as an active ingredient.

Examples of the soyasapogenols include soyasapogenol A, soyasapogenol B, and soyasapogenol C. The at least one soyasapogenol is preferably at least one of soyasapogenol A and soyasapogenol B. In particular, soyasapogenol B is preferred in terms of high selectivity to TGR5. In one embodiment, when soyasapogenol A and soyasapogenol B are used as the soyasapogenols, the ratio of these compounds is not limited. The ratio of soyasapogenol A:soyasapogenol B (weight ratio) may be, for example, 1:99 to 99:1, 10:90 to 90:10, or 10:90 to 30:70.

The soyasapogenols may be obtained from any source and processed by any method. Known plants or parts thereof rich in soyasapogenols include soybeans (in particular, soybean seeds (soybeans)), Azuki beans (binomial name: *Vigna angularis*) (in particular, Azuki bean seeds (Azuki beans)), and Pueraria root. The soyasapogenols can be prepared by extraction and purification from these plants by known methods. The soyasapogenols can also be obtained by hydrolysis of soyasaponins by a known method. Commercially available soyasapogenols can also be used.

In the present invention, a plant-derived material rich in at least one soyasapogenol may be contained in the composition of the present invention as long as the effect of the present invention is obtained. Examples of usable plant-derived materials rich in at least one soyasapogenol include dried fresh soybean seeds or freeze-dried soybean seeds, concentrated or freeze-dried liquids extracted (liquid extracts) from soybean seeds with hot water or an organic solvent, and highly purified soyasapogenols obtained by purifying dried products of liquid extracts by a column or the like. Such plant-derived materials containing at least one soyasapogenol may be commercially available products, or may be prepared from plants such as soybeans by a known method.

Binding of a substance having an activating (agonist) action to TGR5 increases the intracellular cyclic AMP (cAMP) concentration. Transcription of target genes is activated through the increase in cAMP concentration, resulting in various actions. TGR5 activation can be confirmed by evaluating the activity in reporter assay or analyzing gene expression downstream of TGR5 signals.

In one embodiment, the composition for activating TGR5 of the present invention can be used to increase the intracellular cAMP concentration.

It has been reported that activation of TGR5 promoted differentiation of muscle cells and that mice forced to express skeletal muscle-specific TGR5 at high levels showed an increase in muscle mass (Sasaki et al., J. Biol. Chem. (2018) 293(26), 10322-10332). Thus, TGR5 activation increases muscle mass. An increase in muscle mass produces effects such as reduction in muscle strength decline, maintenance of muscle strength, recovery of muscle strength, and improvement of muscle strength.

For example, in skeletal muscle and brown fat cells, TGR5 activation increases mitochondrial activity through an increase in intracellular cAMP concentration, which in turn promotes thermogenesis, improves energy metabolism, and increases fatty acid oxidation. Thus, effects such as promotion of thermogenesis and prevention or reduction of obesity are obtained through TGR5 activation. An increase in muscle mass and an increase in mitochondrial activity are likely to produce effects such as improvement of motor function, improvement of endurance, and reduction in fatigue.

Activation of TGR5 in intestinal endocrine L cells promotes glucose metabolism. Thus, a hypoglycemic effect is obtained through TGR5 activation. It has been reported that bile acid increases gastrointestinal motility through TGR5 activation and acts to alleviate constipation based on the increased gastrointestinal motility (Alemi et al., Gastroenterology. 2013 Jan; 144(1): 145-154.). In addition to the above, it has also been reported that TGR5 activation reduced alcoholic fatty liver (Iracheta-Vellve A et al., Hepatol Commun. 2018 Oct 15; 2(11) :1379-1391) . The composition for activating TGR5 of the present invention is likely to exert the above actions by activating TGR5.

The soyasapogenols are likely to exert the following effects through TGR5 activation: reduction in muscle mass decline, maintenance of muscle mass, recovery of muscle mass, increase in muscle mass, reduction in muscle strength decline, maintenance of muscle strength, recovery of muscle strength, increase in muscle strength, improvement of motor function, improvement of endurance, reduction in fatigue, prevention or improvement of locomotive syndrome or sarcopenia, prevention or reduction of obesity, hypoglycemic effect, promotion of thermogenesis, improvement of energy metabolism, increase in gastrointestinal motility, prevention or alleviation of alcoholic fatty liver, prevention or improvement of metabolic syndrome, and the like.

The composition for activating TGR5 of the present invention can be used, for example, to obtain one or more of the above effects by activating TGR5. In one embodiment, the composition for activating TGR5 of the present invention can be used, for example, for reducing muscle mass decline, maintaining muscle mass, recovering muscle mass, or increasing muscle mass; reducing muscle strength decline, maintaining muscle strength, recovering muscle strength, or increasing muscle strength; or improving motor function or improving endurance. In one embodiment, the composition for activating TGR5 of the present invention can be used for, for example, reducing fatigue, preventing or improving locomotive syndrome or sarcopenia, preventing or reducing obesity, exerting a hypoglycemic effect, promoting thermogenesis, improving energy metabolism, increasing gastrointestinal motility, preventing or alleviating alcoholic fatty liver, or preventing or improving metabolic syndrome.

In the present invention, prevention encompasses preventing the onset of a condition or disease, delaying the onset of a condition or disease, reducing the incidence of a condition or disease, reducing the onset risk of a condition or disease, and the like. Alleviation/improvement/reduction of a condition or disease encompasses helping a subject recover from a condition or disease, alleviating a symptom of a condition or disease, favorably changing a condition or disease, delaying or preventing the progress of a condition or disease, and the like.

The composition for activating TGR5 of the present invention is applicable for both therapeutic use (medical use) and non-therapeutic use (non-medical use).

The composition for activating TGR5 of the present invention can be provided, for example, in the form of a food or beverage, a cosmetic product, a pharmaceutical product, a quasi-pharmaceutical product, feed, or the like, but the composition is not limited thereto. The composition for activating TGR5 of the present invention may be a food or beverage, a cosmetic product, a pharmaceutical product, a quasi-pharmaceutical product, feed, or the like by itself; or may be a preparation or a material such as an additive used in these products. For example, the composition for activating TGR5 of the present invention may be provided in the form of an agent or the like, but it is not limited thereto. The agent may be provided as a composition by itself or as a composition containing the agent.

In one embodiment, the composition for activating TGR5 of the present invention may be an oral composition or a parenteral composition, preferably an oral composition. The oral composition may be a food or beverage, an oral pharmaceutical product, an oral quasi-pharmaceutical product, or feed, preferably a food or beverage. The parenteral composition may be a parenteral pharmaceutical product, a parenteral quasi-pharmaceutical product, or a cosmetic product.

The composition for activating TGR5 of the present invention may contain an optional additive and an optional component in addition to the soyasapogenols, as long as the effect of the present invention is not impaired. The additive and component can be selected according to the form of the composition or the like. Those that can be used generally in food and beverages, cosmetic products, pharmaceutical products, quasi-pharmaceutical products, feed, and the like can be used. Such an optional additive or component may be of one type or a combination of two or more types.

Examples of the optional additive or component include various amino acids and salts thereof such as branched chain amino acids (e.g., valine, leucine, and isoleucine); metabolites of amino acids (e.g., β-hydroxy-β-methylbutyrate (e.g., HMB)) and salts thereof (e.g., HMBCa); proteins such as protein derived from milk (e.g., casein protein and whey protein), protein derived from soybeans, protein derived from rice, protein derived from fish meat, protein derived from wheat, protein derived from peas, collagen; peptides such as imidazole peptides, milk peptides, soybean peptides, and collagen peptides; mucopolysaccharides such as chondroitin sulfate and salts thereof and proteoglycan and salts thereof, or shark cartilage extract containing these mucopolysaccharides; vitamins such as vitamin D, vitamin B1, vitamin B6, vitamin B12, vitamin E, and vitamin C; minerals such as calcium and magnesium; quercetin and a glycoside thereof; procyanidins; polyphenols derived from Gelidiaceae seaweed; walnut polyphenols; grape seed polyphenols; glucosamine and a salt thereof; creatine; folic acid; citric acid; and γ-oryzanol.

Examples of the optional additive or component also include those added to prepare formulations, such as excipients, binders, emulsifiers, isotonic agents, buffering agents, dissolution aids, preservatives, stabilizers, antioxidants, colorants, coagulants, coating agents, and flavoring or masking agents.

When the composition for activating TGR5 of the present invention is provided as a food or beverage, ingredients usable in food and beverages (e.g., food or beverage materials such as the above optional components, and optional food additives) can be added to at least one soyasapogenol to provide various food and beverages. Nonlimiting examples of the food or beverage include general foods and beverages, health foods, foods with function claims, foods for specified health uses, foods and beverages for the sick, food additives, dietary supplements, and raw materials of these products. The food or beverage may be in any form, such as a solid, semi-fluid, or fluid form. The food or beverage may be provided in various dosage forms including solid oral formulations such as tablets, coated tablets, fine granules, granules, powders, pills, capsules, dry syrups, and chewable tablets; and liquid oral formulations such as internal liquid formulations and syrups.

When the composition for activating TGR5 of the present invention is provided as a cosmetic product, a cosmetically acceptable carrier, additive, and the like can be added to at least one soyasapogenol. The cosmetic product may be in any form.

When the composition for activating TGR5 of the present invention is provided as a pharmaceutical product or a quasi-pharmaceutical product, a pharmacologically acceptable excipient and the like can be added to at least one soyasapogenol to provide various dosage forms of pharmaceutical products or quasi-pharmaceutical products. The form of administration of the pharmaceutical product or the quasi-pharmaceutical product may be oral administration or parenteral administration. In order to more sufficiently obtain the effect of the present invention, preferably, the form of administration is oral administration. The dosage form may be a dosage form suitable for administration. Examples of dosage forms of the oral pharmaceutical product or the oral quasi-pharmaceutical product include solid oral formulations such as tablets, coated tablets, fine granules, granules, powders, pills, capsules, dry syrups, and chewable tablets; and liquid oral formulations such as internal liquid formulations and syrups. Examples of dosage forms of the parenteral pharmaceutical product or the parenteral quasi-pharmaceutical product include injection, infusion, ointments, lotions, adhesive skin patches, suppositories, nasal agents, and transpulmonary agents (inhalants). The pharmaceutical product may be for non-human animals.

When the composition for activating TGR5 of the present invention is provided as feed, a component usable in feed can be added to at least one soyasapogenol to provide the feed. Examples of the feed include livestock feed for animals such as cows, pigs, chickens, sheep, and horses; feed for small animals such as rabbits, guinea pigs, rats, and mice; and pet food for animals such as dogs, cats, and birds.

When the composition for activating TGR5 of the present invention is provided as a food or beverage, a cosmetic product, a pharmaceutical product, a quasi-pharmaceutical product, feed, or the like, its production method is not limited. Such a product can be produced by a usual method, using at least one soyasapogenol.

In the production of the composition for activating TGR5 of the present invention, examples of usable soyasapogenols include purified compounds and the above-described plant-derived materials rich in at least one soyasapogenol. The soyasapogenols may be added in the form of plant-derived materials containing the compounds.

A package, container, package insert, or the like of the composition for activating TGR5 of the present invention may include one or more descriptions regarding usage, types of active ingredients, the above-described effects, instructions for use (e.g., ingestion method or administration method), and the like. The composition for activating TGR5 of the present invention may be labeled as having a TGR5-activating action or an action based on the TGR5-activating action. In one embodiment, the composition for activating TGR5 of the present invention may be labeled with one or more function claims such as "reduction in muscle decline", "maintenance of muscle", "increase in muscle", "improvement of muscle", "reduction in muscle strength decline", "maintenance of muscle strength", " increase in muscle strength", "improvement of muscle strength", "muscle building support", "improvement of walking function", "maintenance of walking function", "improvement of motor function", "maintenance of motor function", "maintenance of muscle that weakens with aging", and "maintenance of muscle strength that weakens with aging".

In one embodiment, the composition for activating TGR5 of the present invention may be labeled with one or more function claims such as "anti-obesity", "prevention of obesity", "reduction in obesity", "reduction in waist circumference", "maintenance of waist circumference", "maintenance of lean body", "reduction in body fat accumulation", "reduction in body fat", "reduction in visceral fat accumulation", "reduction in visceral fat", "reduction in fat accumulation in the liver", "fat reduction in the liver", "weight loss", "reduction in weight", "diet", "fat burning", "fat consumption", "lipid metabolism", "mitochondrial function", "thermogenesis", "basal metabolism", "metabolic function", "metabolic capacity", "prevention of metabolic syndrome", and "improvement of metabolic syndrome".

In another embodiment, the composition for activating TGR5 of the present invention may be labeled with one or more function claims such as "reducing an increase in postprandial blood glucose levels", "moderating an increase in postprandial blood glucose levels", "reducing blood glucose levels", "for those who are concerned about blood glucose levels", "for those who are concerned about postprandial blood glucose levels", "reducing the predisposition for hyperglycemia".

The amount of soyasapogenols in the composition for activating TGR5 of the present invention can be appropriately set according to the form of the composition or the like. For example, the total amount of soyasapogenols in the composition may be 0.0001 to 95 wt%, 0.001 to 90 wt%, or 0.01 to 90 wt%. In one embodiment, when the composition for activating TGR5 of the present invention is provided as an oral composition such as a food or beverage, a pharmaceutical product, or a quasi-pharmaceutical product, the total amount of soyasapogenols in the composition is preferably 0.01 wt% or more, more preferably 0.2 wt% or more, and is preferably 20 wt% or less, more preferably 10 wt% or less. In one embodiment, the total amount of soyasapogenols in the composition for activating TGR5 of the present invention is preferably 0.01 to 20 wt%, more preferably 0.2 to 10 wt%. When the composition contains two or more soyasapogenol compounds, the total amount is the sum of these compounds. The amount of soyasapogenols can be measured according to a known method, such as HPLC.

The composition for activating TGR5 of the present invention can be ingested or administered by a method appropriate to the form. The composition for activating TGR5 of the present invention is preferably orally administered or orally ingested.

The intake (dosage) of the composition for activating TGR5 of the present invention is not limited as long as it is an amount (effective amount) that produces the TGR5 activation effect. The intake may be appropriately set according to the form of administration, administration method, body weight, and the like. For example, when a human (adult) is subjected to oral administration or injection, the total daily intake of soyasapogenols is preferably 5 mg or more, more preferably 10 mg or more, still more preferably 20 mg or more, and is also preferably 500 mg or less, more preferably 200 mg or less, still more preferably 100 mg or less. In one embodiment, when a human (adult) is subjected to oral administration or ingestion, the total daily intake of soyasapogenols is preferably 5 to 500 mg, more preferably 10 to 200 mg, still more preferably 20 to 100 mg. The above amount of soyasapogenols is preferably orally administered or ingested, for example, once per day or in two or three portions per day. When a human (adult) is subjected to ingestion of the composition for activating TGR5 of the present invention in order to obtain the TGR5 activation effect, preferably, a subject is orally fed or administered with the composition of the present invention in such a manner that the total intake of soyasapogenols per 60 kg per day is in the above ranges.

In one embodiment, preferably, the composition for activating TGR5 of the present invention contains the soyasapogenols in an amount that can produce the desired effect of the present invention, i.e., the effective amount, taking into consideration its form, administration method, and the like. In one embodiment, for example, when the composition for activating TGR5 of the present invention is an oral composition such as a food or beverage or an oral pharmaceutical product, the total amount of soyasapogenols in the intake per 60 kg adult per day is preferably 5 to 500 mg, more preferably 10 to 200 mg, still more preferably 20 to 100 mg.

A subject (hereinafter also simply referred to as an "administration subject) to be administered or fed with the composition for activating TGR5 of the present invention is preferably a mammal (a human or non-human mammal), more preferably a human. In the present invention, the administration subject is preferably a subject who needs or wants to activate TGR5. In one embodiment of the present invention, the administration subject may be a subject who needs or wants to increase muscle mass, reduce muscle mass decline, maintain muscle mass, or recover muscle mass; a subject who needs or wants to increase muscle strength, reduce muscle strength decline, maintain muscle strength, or improve muscle strength; or a subject who needs or wants to improve motor function or improve endurance.

The present invention also encompasses the following method of activating TGR5 and use for activating TGR5:
a method of activating TGR5, including administering at least one soyasapogenol, and
use of at least one soyasapogenol for activating TGR5.

The method may be therapeutic or non-therapeutic. The use may be therapeutic or non-therapeutic.

Administering at least one soyasapogenol to a subject can activate TGR5 in the subject. The at least one soyasapogenol can be used for activating TGR5 in a subject.

In the above method and use, the soyasapogenols are simply used in an amount (effective amount) that produces a desired action. The soyasapogenols, subject, administration method, dosage, preferred embodiments thereof, and the like are as described above for the composition for activating TGR5. The soyasapogenols may be directly administered, or may be administered in the form of a composition containing the soyasapogenols. For example, the above-described composition for activating TGR5 of the present invention may be administered.

The present invention also encompasses use of at least one soyasapogenol for producing a composition for activating TGR5. The composition for activating TGR5 and preferred embodiments thereof are as described above.

All academic literature and patent literature cited herein are incorporated herein by reference.

### EXAMPLES

The present invention is described in further detail below with reference to examples, but the scope of the present invention is not limited thereto.

### <Example 1>

The TGR5-activating action of a test substance was evaluated by luciferase assay.

This evaluation system determines activation of human TGR5 (hTGR5) based on transcription activation of cAMP response element binding protein (CREB) which is a downstream signal of TGR5. An increase in intracellular cAMP concentration activates binding of CREB to CRE (cAMP-responsive element) and induces transcription of a luciferase gene. This makes it possible to evaluate CREB activation by quantifying luciferase activity.

In order to evaluate selectivity of the test substance to TGR5, luciferase assay was performed not only on cells forced to express hTGR5 (also referred to as hTGR5-forced expression cells) but also on cells forced to express glucagon-like peptide-1 (GLP-1) receptor (GLP1R), which is a G protein-coupled receptor as with hTGR5, instead of hTGR5 (such forced cells are also referred to as hGLP1R-forced expression cells). The hGLP1R-forced expression cells are not expressing hTGR5.

GLP1R and TGR5 are both G protein-coupled receptors. Activation of GLP1R increases intracellular cAMP. When addition of a test substance increases luciferase activity in hTGR5-forced expression cells, the substance is determined as having a TGR5-activating action. When addition of a test substance to the hTGR5-forced expression cells and the hGLP1R-forced expression cells result in a greater increase in luciferase activity in the hTGR5-forced expression cells than in luciferase activity in the hGLP1R-forced expression cells, the test substance is considered to be more selective to TGR5.

### (Construction of expression vector)

A hTGR expression vector (pTGR5) was constructed by the following procedure.

With referring to NM _001077191 as the base sequence of a human-derived TGR5 gene, an artificial gene encoding the human TGR5 was designed to synthesize DNA. The synthesized DNA was inserted into an expression vector pcDNA3.1(+) for eukaryotes (Invitrogen^{®}), Thermo Fisher Scientific), using cloning sites BamHI and EcoRI of the vector, whereby a hTGR expression vector (plasmid) was constructed. This plasmid was produced using E. coli K12 (dam+ dcm+ tonA) as the host of transformation. The constructed hTGR expression vector was named pTGR5.

pGL4.29 [luc2P/CRE/Hygro] available from Promega was used as a CRE-reactive luciferase expression vector (pCRE/Luc). This plasmid is a vector that holds a luciferase gene linked to cAMP response element (CRE).

Two plasmids pTGR5 and pCRE/Luc were introduced into E. coli JM109. Then, the transformed E. coli was cultured, and plasmids in an amount sufficient for an experiment were purified. The purified plasmids and the plasmid used for transformation were treated with multiple restriction enzymes and electrophoresed to compare the enzyme-treated fragments and identify identical patterns, whereby it was confirmed that the structure of the purified plasmids was kept identical to that of the original vector (the results are not shown). The purified plasmids were used in the following experiment.

### (Production of hTGR-forced expression cells)

In the following test, high-glucose Dulbecco's Modified Eagle Medium (DMEM) (FUJIFILM Wako Pure Chemical Corporation) was used as DMEM. Phosphate buffered salts (PBS) available from Takara Bio Inc. were used.

HEK293 cells (human fetal kidney cell line, ATCC:CRL-2828) were subcultured in DMEM-10% FBS medium (DMEM containing 10% fetal bovine serum (FBS) (Funakoshi Co., Ltd.)) in a T75 flask. The medium was removed, and the cells were washed with a PBS(-) solution (10 mL). A trypsin solution (0.25% trypsin-EDTA (NACALAI TESQUE, INC.) diluted 10 times with a PBS solution) (1 mL) was added to the cells and left standing for about 30 seconds. Then, the cells were scraped, and the medium (10 mL) was added to recover the cells. The recovered cells were transferred to a 50 mL centrifuge tube and centrifuged at 1000 rpm at room temperature for three minutes. The resulting pellets were loosened in the medium (2 mL), and a part of the pellets was diluted 10 times with 0.2% Trypan blue. The number of cells was counted using a hemocytometer. The medium and the cell suspension were dispensed into a new T75 flask to obtain a cell count of 6 × 10⁶ cells/25 mL, followed by culturing in an incubator (37°C, 5% CO₂) for about 22 hours. These cells were used to introduce the plasmids thereinto.

The plasmids were introduced into the HEK293 cells by the following procedure.

X-tremeGENE^{®} (Roche) (45 µL) was placed in a 1.5 mL tube. OptiMEM^{®} I Reduced Serum Medium (Thermo Fisher Scientific) (705 µL) at room temperature was added thereto. After quick mixing by inversion twice, the liquid mixture was spun down, whereby an X-tremeGENE-diluted solution was obtained. After standing for five minutes at room temperature, the plasmids (hTGR5 expression vector (pTGR) (5 µg) and the CRE-reactive luciferase expression vector (pCRE/Luc) (5 µg)) were added to the X-tremeGENE-diluted solution (750 µL). After quickly mixing by inversion twice, the mixture was spun down and left standing at room temperature for 25 minutes. During this 25 minutes, the medium in the F75 flask in which the HEK293 cells were seeded as described above was replaced with DMEM + 5% FBS (without antibiotics) (15 mL). After standing at room temperature for 25 minutes, the X-tremeGENE-diluted solution (765 µL) containing the plasmids was quietly added (dropwise) to the HEK293 cells in the flask, followed by gentle mixing and culturing under conditions of 37°C and 5% CO₂ for five hours. The medium was removed, and the cells were washed with a PBS(-) solution (10 mL). A trypsin solution (0.25% trypsin-EDTA diluted 10 times with a PBS solution) (1 mL) was added to the cells and left standing for about 30 seconds. Then, the cells were scraped, and the medium (10 mL) was added to recover the cells. The revered cells were transferred to a 50 mL centrifuge tube and centrifuged at 1000 rpm at room temperature for three minutes. The resulting pellets were loosened in the medium (2 mL), and a part of the pellets was diluted 10 times with 0.2% Trypan blue. The number of cells was counted using a hemocytometer. The cells were suspended in a seed medium (DMEM (FBS-free, containing penicillin/streptomycin, FUJIFILM Wako Pure Chemical Corporation)). Then, the cells were seeded into a 96-well white clear plate at 75 µL/well (Corning, Corning 96-well Clear Flat Bottom Polystyrene TC-treated Microplates, white), and cultured in an incubator (37°C, 5% CO₂), whereby hTGR-forced expression cells were obtained.

### (Production of hGLP1-forced expression cells)

A vector that expresses a human GLP1 receptor (hGLP1R) gene was introduced into the HEK293 cell to produce a cell forced to express hGLP1R.

GLP1R (NM_002062) Human Untagged Clone (ORIGENE) was used as a hGLP1R expression vector. This expression vector contains a hGLP1R gene inserted into a vector pCMV6-XL5, and has a structure that expresses hGLP1R.

The hGLP1R expression vector was introduced into the HEK293 cell in the same manner as described above, except that the hGLP1R expression vector was used instead of the hTGR5 expression vector (pSTW01). Thus, hGLP1R-forced expression cells were obtained.

### (Test substance)

Soyasapogenol A and soyasapogenol B were used as the test substances. Soybean saponin (Saponin B-50 available from J-Oil Mills, Inc.) was also used for comparison.

Soyasapogenol A and soyasapogenol B were prepared by the following method. Saponin B-50 (available from J-Oil Mills, INC.) was reacted in a 2N aqueous hydrochloric acid solution in an amount 20 times that of Saponin B-50 at 100°C for two hours to cleave a sugar chain of the saponin. After completion of the reaction, the reaction solution was cooled to room temperature, and neutralized with an aqueous sodium hydroxide solution. The reaction solution was suction filtered, the residue was washed with a large amount of distilled water, and suction filtration was repeated until there was no remaining sodium hydroxide. The resulting product was dried, whereby a soyasapogenol coarse fraction (purity 50%: a total of soyasapogenol A and soyasapogenol B) was obtained. This soyasapogenol coarse fraction was further purified by column chromatography using a mixture of silica gel, hexane, and ethyl acetate, whereby soyasapogenol A (purity 99% or higher) and soyasapogenol B (purity 99% or higher) were obtained. The resulting soyasapogenol A and soyasapogenol B were used.

### (Measurement of luciferase activity)

The hTGR-forced expression cells were seeded and cultured for 24 hours. Thereafter, the test substance was added (final concentration: 20 µg/mL), followed by culturing for additional five hours. The test substance (soyasapogenol A, soyasapogenol B, or soybean saponin) was added in the form of a solution in dimethyl sulfoxide (DMSO). In addition, DMSO was added instead of the test substance to provide a negative control (final concentration: 0.1%). After culturing, Dual-Glo Luciferase Assay Reagent included in Dual-Glo^{®} Luciferase Assay System (Promega) was added in an amount of 75 µL/well, and the resulting mixture was kept warm at room temperature for 10 minutes. Then, the emission intensity was measured using a multimode microplate reader "FlexStation 3" (Molecular Devices). The emission intensity was regarded as the luciferase activity. In this evaluation system, Renilla reniformis luciferase was used as the internal standard.

In order to evaluate the selectivity to hTGR5, luciferase luminescence was measured in the same manner as described above, using the hGLP1R-forced expression cells instead of the hTGR5-forced expression cells.

Lithocholic acid (Tokyo Chemical Industry Co., Ltd.) instead of the test substance was dissolved in DMSO and added to each of the hTGR5-forced expression cells and the hGLP1R-forced expression cells (final concentration: 3.3 µM) to provide a positive control. The emission intensity was measured by the above method. As another positive control, Taurolithocholic Acid Sodium Salt (FUJIFILM Wako Pure Chemical Corporation) was dissolved in DMSO, and added to each of the hTGR5-forced expression cells and the hGLP1R-forced expression cells (final concentration: 3.3 µM). The emission intensity was measured by the above method. Each of the positive controls of the hTGR5-forced expression cells and the hGLP1R-forced expression cells containing either lithocholic acid or Taurolithocholic Acid Sodium Salt showed an emission intensity higher than that of the corresponding negative control wherein DMSO was added.

### (Results)

### (TGR5-activating action)

The hTGR5-forced expression cells to which soyasapogenol A, soyasapogenol B, or soybean saponin was added showed a significantly higher emission intensity than the negative control. This indicates that soyasapogenol A, soyasapogenol B, and soybean saponin have a hTGR5-activating action.

### (Selectivity to hTGR5)

The ratio of emission intensity of the hTGR5-forced expression cells to which the test substance was added to the emission intensity of the negative control in which DMSO was added to the hTGR5-forced expression cells (emission intensity of the cells to which the test substance was added/emission intensity of the negative control) was regarded as the luciferase activity (Luc-hTGR5) of the hTGR5-forced expression cells to which the test substance was added.

The ratio of emission intensity of the hGLP1R-forced expression cells to which the test substance was added to the emission intensity of the negative control in which DMSO was added to the hGLP1R-forced expression cells (emission intensity of the cells to which the test substance was added/emission intensity of the negative control) was regarded as the luciferase activity (Luc-hGLP1R) of the hGLP1R-forced expression cells to which the test substance was added.

The selectivity of the test substance to TGR5 was evaluated based on the ratio (Luc-hTGR5/Luc-hGLP1R) of luciferase activity (Luc-hTGR5) of the hTGR5-forced expression cells to luciferase activity (Luc-hGLP1R) of the hGLP1R-forced expression cells to which the test substance was added.

Table 1 shows the ratio (Luc-hTGR5/Luc-hGLP1R) of luciferase activity (Luc-hTGR5) of the hTGR5-forced expression cells to luciferase activity (Luc-hGLP1R) of the hGLP1R-forced expression cells to which the test substance was added. A higher luciferase activity ratio (Luc-hTGR5/Luc-hGLP1R) indicates a higher selectivity to hTGR5.

**[Table 1]**

| | **Luciferase activity ratio (Luc-hTGR5/Luc-hGLP1R)** |
|---|---|
| **Soyasapogenol A** | **1.60** |
| **Soyasapogenol B** | **2.88** |
| **Soybean saponin** | **0.13** |

The above shows that soyasapogenol A and soyasapogenol B have a TGR5-activating action. In addition, soyasapogenol A and soyasapogenol B showed a significantly higher luciferase activity ratio (Luc-hTGR5/Luc-hGLP1R) than soybean saponin. This shows that soyasapogenol A and soyasapogenol B are more selective to TGR5 than soybean saponin.

The following describes formulation examples to prepare the composition for activating TGR5 of the present invention, but the present invention is not limited thereto. In the formulation examples, examples of usable soyasapogenols include a mixture of soyasapogenol A and soyasapogenol B, soyasapogenol A, and soyasapogenol B. The mixture of soyasapogenol A and soyasapogenol B which can be used may be, for example, a mixture of soyasapogenol A and soyasapogenol B at a weight ratio of 1:99 to 99:1. The following formulation examples are merely examples, and for example, different proteins can be used instead of the following proteins. Likewise, different peptides, amino acids, and the like can be used instead of the following peptides, amino acids, and the like.

### <Formulation Example 1> Tablet

Soyasapogenol 67 g
Glucosamine 500 g
Shark cartilage extract 167 g
Sucrose fatty acid ester 90 g
Silicon oxide 90 g
Starch 87 g

These components are mixed together and tableted using a single-shot tableting machine, whereby tablets each having a diameter of 9 mm and a mass of 300 mg are produced.

### <Formulation Example 2> Tablet

Soyasapogenol 67 g
Branched chain amino acid (BCAA) 333 g
β-Hydroxy-β-methylbutyrate (HMB) calcium 133 g
Sucrose fatty acid ester 90 g
Silicon oxide 90 g
Starch 253 g
Vitamin D 0.03 g
Proteoglycan 33 g

These components are mixed together and tableted using a single-shot tableting machine, whereby tablets each having a diameter of 9 mm and a mass of 300 mg are produced.

### <Formulation Example 3> Granule

Soyasapogenol 5 g
Soybean protein 350 g
Milk protein 350 g
Soybean peptide 50 g
Milk peptide 50 g
Quercetin glycoside 5 g
Grape seed polyphenol 5 g
Dextrin 154 g
Thickener 30 g
Sucralose 0.5 g
Flavoring or masking agent 0.5 g

The above powder materials are uniformly mixed, and then a 10% hydroxypropyl cellulose-ethanol solution (800 mL) is added to the powder mixture, followed by kneading, extrusion, and drying by usual methods, whereby granules are obtained.

### <Formulation Example 4> Health drink

Disodium DL-tartrate 0.10 g
Succinic acid 0.01 g
Liquid sugar 800.00 g
Citric acid 12.00 g
Vitamin C 10.00 g
Soyasapogenol 0.50 g
Collagen 40.00 g
Cyclodextrin 5.00 g
Emulsifier 5.00 g
Flavoring or masking agent 15.00 g
Potassium chloride 1.00 g
Magnesium sulfate 0.50 g

The above components are mixed, and water was added to the mixture to make up 1 liter. This drink is consumed in an amount of 100 mL or more per time.

## Claims

1. A composition for activating TGR5, comprising:
at least one soyasapogenol as an active ingredient.

2. The composition for activating TGR5 according to claim 1, wherein the at least one soyasapogenol is at least one of soyasapogenol A and soyasapogenol B.

3. The composition for activating TGR5 according to claim 1 or 2, wherein the composition is a food or beverage, a cosmetic product, or a quasi-pharmaceutical product.

4. Use of at least one soyasapogenol for activating TGR5.

5. A method of activating TGR5, comprising:
administering at least one soyasapogenol.
